# EUROPÄISCHE PATENTSCHRIFT

(11) **EP 1 041 926 B1**
(45) Veröffentlichungstag und Bekanntmachung des Hinweises auf die Patenterteilung: **23.06.2004**
(21) Anmeldenummer: 98966401.6
(22) Anmeldetag: 23.12.1998
(51) Int. Cl.: A61B 5/0225

(54) **BLUTDRUCKMESSGERÄT**
DEVICE FOR MEASURING BLOOD PRESSURE
APPAREIL POUR MESURER LA TENSION ARTERIELLE

(30) Priorität: 24.12.1997 DE 19757973
(43) Veröffentlichungstag der Anmeldung: 11.10.2000
(73) Patentinhaber: Braun GmbH, 61476 Kronberg (DE)
(72) Erfinder: FREUND, Dirk, D-65779 Kelkheim (DE); SCHNAK, Fred, D-61476 Kronberg (DE); GIERSIEPEN, Martin, D-61440 Oberursel (DE); KRESSMANN, Frank, D-65824 Schwalbach (DE); HARTTMANN, Brigitte, D-65527 Niedernhausen (DE)
(86) Internationale Anmeldenummer: PCT/EP1998/008430
(87) Internationale Veröffentlichungsnummer: WO 1999/033396

(56) Entgegenhaltungen:
- US-A- 4 178 918
- US-A- 5 368 039

## Beschreibung

Die vorliegende Erfindung betrifft ein Blutdruckmeßgerät mit einer fluidbefüllbaren Manschette zum Anlegen an ein Körperglied, einer Befüllvorrichtung zum Befüllen der Manschette mit Fluid, einer Auslaßvorrichtung zum Auslassen des Fluids aus der Manschette und einem Drucksensor der derart in oder an die Manschette gekoppelt ist, daß Druckoszillationen zur oszillometrischen Bestimmung des Blutdruckes meßbar sind und wobei die Auslaßvorrichtung ein antreibbares Fördermittel zum Abfördem des Fluids aus der Manschette aufweist.

Aus der US-A-4,178,918 ist bereits ein Blutdruckmeßgerät der eingangs genannten Art bekannt.

Oszillometrisch arbeitende Blutdruckmeßgeräte weisen eine flexible Manschette auf, die an einem Körperglied, wie beispielsweise am Oberarm, am Handgelenk oder an einem Finger, angelegt wird. Die Manschette besitzt einen Innenraum, der mit Luft oder einem anderen Fluid gefüllt werden kann.

Zur Messung des arteriellen Butdrucks wird die Manschette aufgepumpt und der Luftdruck in der Manschette derart erhöht, daß die Arterie bis zur vollständigen Undurchlässigkeit zusammengedrückt wird. Anschließend wird das Fluid aus der Manschette langsam abgelassen und der Fluiddruck kontinuierlich wieder auf Atmosphärendruck reduziert. Dabei tritt durch den Puls eine Oszillation des Fluiddrucks in der Manschette auf, aus der sich der systolische und der diastolische Blutdruckwert bestimmen läßt. Sobald der Fluiddruck soweit abgesenkt ist, daß keine Oszillationen mehr auftreten, wird der Manschettendruck in der Regel rasch reduziert, um den Meßvorgang zu verkürzen. Übliche Werte für das langsame Ablassen während der Messung sind Druckänderungen von 1 bis 5 mmHg/s. Der Manschettendruck wird dabei über eine Druckmeßvorrichtung erfaßt.

Das Aufpumpen bzw. Befüllen der Manschette erfolgt in der Regel mit einer Pumpe, die hand- oder motorbetätigt sein kann. Um den Ablaßvorgang regeln zu können, besitzt die Auslaßvorrichtung in der Regel ein oder mehrere Ventile. Der Ablaßvorgang ist dabei in zwei Phasen unterteilt, nämlich in eine erste Phase, in der der Druck langsam abgesenkt wird, um die Oszillationen exakt erfassen zu können, und eine zweite Phase, in der der Druck rasch abgesenkt wird, um nach dem Auftreten der Oszillationen den Patienten möglichst schnell zu entlasten. Dabei können entweder beide Druckabsenkgeschwindigkeiten mit einem Ablaßventil realisiert sein oder die Auslaßvorrichtung kann für das langsame Absenken des Manschettendrucks ein erstes Ablaßventil und für das rasche Absenken des Manschettendrucks ein zweites Ablaßventil aufweisen. Um das langsame Absenken des Manschettendrucks exakt steuern zu können und eine möglichst konstante Druckabsenkrate zu erreichen, sind in der Regel teuere Stromregelventile notwendig. Für die langsame Druckabsenkung sind bereits verschiedene Ablaßventile vorgeschlagen worden (vgl. DE 43 09 783 C2, EP 0 576 328 A1, EP 0 335 179 A1), die jedoch alle verhältnismäßig teuer sind.

Auf das teuere Ablaßventil für das langsame Druckablassen kann verzichtet werden, wenn die Druckoszillationen während des Aufpumpens bzw. Befüllens der Manschette detektiert wird. Hierzu muß jedoch mit einer regelbaren Pumpe die Manschette sehr langsam aufgepumpt werden, um die Oszillationen erfassen zu können. Dieses langsame, aber stetige Anwachsen des Manschettendrucks wird von den Benutzern jedoch als sehr unangenehm empfunden.

Der vorliegenden Erfindung liegt daher die Aufgabe zugrunde, ein verbessertes Blutdruckmeßgerät zu schaffen, daß die Nachteile bekannter Blutdruckmeßgeräte vermeidet. Insbesondere soll das zu schaffende Blutdruckmeßgerät mit einem einfachen Aufbau eine Blutdruckmessung mit hoher Genauigkeit ermöglichen.

Erfindungsgemäß wird diese Aufgabe bei einem Blutdruckmeßgerät mit den Merkmalen des Anspruchs 1 gelöst. Die Druckverminderung in der Manschette wird nicht durch ein bloßes Ablassen des druckbeaufschlagten Fluids bewirkt, sondern durch ein aktives Wegfördern durch ein energiegetriebenes Fördermittel, insbesondere eine Pumpe. Hierdurch kann die Fluidmenge in der Manschette um eine genau bestimmbare Menge pro Zeiteinheit vermindert werden, um die gewünschte Druckabsenkrate zu erreichen und während des Absenkens die Druckoszillationen zu erfassen.

Das Blutdruckmeßgerät ist frei von Stromregelventilen. Gegebenenfalls kann auf der Ablaßseite der Manschette ein Schaltventil für das schnelle Druckabsenken am Ende des Meßvorganges vorgesehen sein. Vorzugsweise jedoch wird die Druckabsenkung alleine durch das antreibbare Fördermittel gesteuert. Durch den Verzicht auf ein teueres Stromregelventil ist der Aufbau des Blutdruckmeßgerätes vereinfacht und dementsprechend kostengünstig.

Erfindungsgemäß ist das Fördermittel gemeinsam sowohl für die Befüllvorrichtung als auch für die Auslaßvorrichtung vorgesehen. Die Druckerhöhung und die Druckabsenkung bzw. das Befüllen und das Ablassen der Manschette wird mit demselben Fördermittel bewirkt. Ein einziges Fördermittel ist also ausreichend. Als Fördermittel ist vorzugsweise eine Pumpe mit regelbarem oder steuerbarem Durchsatz vorgesehen.

Um mit dem Fördermittel den Druck in der Manschette sowohl erhöhen als auch absenken zu können, ist eine Umkehrvorrichtung zur Umkehrung der Förderrichtung des Fördermittels vorgesehen. Die Umkehrvorrichtung ermöglicht es, von einer ersten Förderrichtung zu einer zweiten entgegengesetzten Förderrichtung und umgekehrt umzuschalten.

In einer nicht beanspruchten Weiterbildung ist das Fördermittel selbst umschaltbar, d. h. das Fördermittel besitzt zwei Förderrichtungen und ist in entgegengesetzten Förderrichtungen antreibbar ausgebildet. Um ein einfaches Umschalten von Aufpumpen zu Abpumpen zu ermöglichen, kann vorteilhafterweise ein rotatorisch arbeitender Elektroantrieb für das Fördermittel vorgesehen sein, wobei der Elektroantrieb eine Umpoleinrichtung zur Umkehr der Antriebs- bzw. Förderrichtung aufweist. Zweckmäßigerweise ist als Antrieb ein Gleichstrommotor mit einem umpolbaren Energieanschluß vorgesehen.

Erfindungsgemäß ist ein Fördermittel mit einer Förderrichtung vorgesehen sein. In der Fluidverbindung zwischen dem Fördermittel und der Manschette ist eine Schaltvorrichtung vorgesehen, wobei die Schaltvorrichtung eine erste Schaltstellung, in der die Saugseite des Fördermittels mit der Manschette fluidverbunden ist, und eine zweite Schaltstellung aufweist, in der die Pumpseite des Fördermittels mit der Manschette fluidverbunden ist. Eine derartige Schaltvorrichtung ermöglicht die Verwendung einer kostengünstigen unidirektionalen Pumpe.

Vorzugsweise weist die Schaltvorrichtung zwei Schaltventile auf, die bezüglich der Förderrichtung auf verschiedenen Seiten des Fördermittels angeordnet sind und die derart miteinander gekoppelt sind, daß stets nur entweder die Pumpseite oder die Saugseite des Fördermittels mit der Manschette in Fluidverbindung steht. In ihrer Ruhestellung sind die Schaltventile vorzugsweise derart geschaltet, daß ein Ablassen des Fluids aus der Manschette möglich ist. Hierdurch kann auch ohne den Betrieb des Fördermittels das Fluid aus der Manschette abgelassen werden.

Vorzugsweise sind beide Schaltventile mit einer gemeinsamen Manschettenleitung verbunden. Durch die gemeinsame Manschettenleitung ist sowohl Fluid in die Manschette pumpbar als auch Fluid aus der Manschette ablaßbar. Dies bewirkt kurze Fluidleitungen zwischen der Manschette und dem Fördermittel und ein dementsprechend geringes Totvolumen in den Leitungen. Die Druckerhöhung bzw. -senkung kann daher schnell und genau bewirkt werden.

In Weiterbildung der Erfindung ist eine Steuereinheit zur Steuerung des Fördermittels vorgesehen. Bei dem Fördermittel können unterschiedliche Förderraten bzw. -geschwindigkeiten eingestellt werden, so daß unterschiedliche Druckabsenkraten erzielt werden können. Vorzugsweise ist die Steuereinheit mit einer Druckerfassungseinheit zur Erfassung des Fluiddrucks in der Manschette verbunden und zur Steuerung des Fördermittels in Abhängigkeit des erfaßten Fluiddrucks ausgebildet.

Die Steuerung kann dabei derart ausgebildet sein, daß eine Druckminderung, insbesondere das langsame Ablassen des Fluids zur Erfassung der Druckoszillationen, durch Undichtigkeiten der Auslaßvorrichtung, insbesondere der Pumpe, bewirkt, bzw. unterstützt wird. Eine exakte Druckabsenkrate kann dabei durch das Zu- und Abfördern von Fluid durch das Fördermittel gesteuert werden. Die Steuerung der Druckabsenkung in Abhängigkeit des tatsächlich erfaßten Manschettendrucks ermöglicht es, einen vorgegebenen Verlauf der Druckabsenkung exakt einzuhalten.

Entsprechend einer vorteilhaften Ausgestaltung der Erfindung ist das Fördermittel von der Steuereinheit derart ansteuerbar, daß der Fluiddruck in der Manschette zumindest in einer Phase des Ablaßvorganges mit einer konstanten Druckabfallrate vermindert wird, insbesondere derart, daß der Fluiddruck in einer ersten Phase des Ablaßvorganges mit einer ersten niedrigen, im wesentlichen konstanten Druckabfallrate und in einer zweiten Phase mit einer zweiten hohen Druckabfallrate vermindert wird. Die schnelle Absenkung des Fluiddrucks in der zweiten Phase kann dabei gegebenenfalls durch das Öffnen eines Schaltventiles in einer mit der Manschette verbundenen Fluidleitung bewirkt werden. Vorzugsweise aber wird der rasche Druckabfall durch eine erhöhte Abförderleistung des Fördermittels bewirkt. In diesem Fall kann auch in der zweiten Phase die Druckabfallrate exakt eingestellt werden und auf ein entsprechendes Schaltventil verzichtet werden.

Vorteilhafterweise wird der Aufpump- bzw. Ablaßvorgang indirekt durch den Betriebszustand bzw. den Widerstand des Fördermittels erfaßt. Insbesondere kann als Ablaß-Erfassungsvorrichtung ein Strommesser zur Messung eines elektrischen Eingangsstromes des Fördermittels vorgesehen sein. Am Ende des Ablaßvorganges erfolgt ein Stromanstieg. Die Steuereinheit kann in Abhängigkeit eines entsprechenden Signales des Strommessers das Fördermittel abschalten.

Eine vorteilhafte Ausführung der Erfindung besteht auch darin, daß eine Störfiltervorrichtung zur Filterung von Störungen in dem Drucksignal der Manschettendruck-Erfassungsvorrichtung vorgesehen ist. Je nach Typ des verwendeten Fördermittels kann es zu Schwankungen des Manschettendrucks durch Volumenpulsation des Fördermittels kommen. Für Schwankungen einer Größe, die sich störend auf die Auswertung des Manschettendrucks auswirken, ist eine Filterung vorgesehen. Eine geeignete Filterung kann grundsätzlich mit elektronischen oder mechanischen Mitteln erfolgen.

Gemäß einer bevorzugten Ausführung besitzt die Störfiltervorrichtung eine Erfassungsvorrichtung zur Erfassung der Betriebsfrequenz des Fördermittels und eine Frequenzfiltereinrichtung zur Filterung von Signalanteilen des Manschettendrucksignales mit einer der Betriebsfrequenzen entsprechenden Frequenz. Die Filterung dieser Signalanteile kann elektronisch, z. B. analog oder in einem Microcontroller bewirkt werden.

Vorzugsweise kann die Störfiltervorrichtung auch zwischen dem Fördermittel und der Erfassungsvorrichtung zur Erfassung des Manschettendrucks ein mechanisches Dämpferglied zur Dämpfung von Druckschwankungen im Fluid aufweisen. Vorzugsweise ist das Dämpferglied auf die Betriebsfrequenz des Fördermittels abgestimmt.

Vorzugsweise wird die Filterung also in der Weise bewirkt, daß die Betriebsfrequenz des Fördermittels erfaßt wird und Störungen auf dem Signal der Erfassungsvorrichtung für den Manschettendruck mit der gleichen Frequenz wie die Betriebsfrequenz des Fördermittels mit einer geeigneten Filterung eliminiert wird. Die Störfiltervorrichtung ermöglicht die Erfassung der Druckoszillationen infolge des Pulses mit einer erhöhten Genauigkeit.

Die Erfindung wird nachfolgend anhand von Ausführungsbeispielen und zugehöriger Zeichnungen näher erläutert. In diesen zeigen:
- Figur 1: ein Blutdruckmeßgerät gemäß einer nicht tnanspruchten Ausführung in einer schematischen Darstellung, wobei als Fördermittel eine Pumpe mit zwei Förderrichtungen vorgesehen ist,
- Figur 2: ein Blutdruckmeßgerät gemäß einer Ausführung der Erfindung in einer schematischen Darstellung ähnlich Figur 1, wobei als Fördermittel eine Pumpe mit einer Förderrichtung und eine Strömungs-Umkehrvorrichtung zur Umkehrung der Förderrichtung vorgesehen ist, und
- Figur 3: ein Diagramm, das den Verlauf des Manschettendrucks über der Zeit bei einem Meßvorgang zeigt.

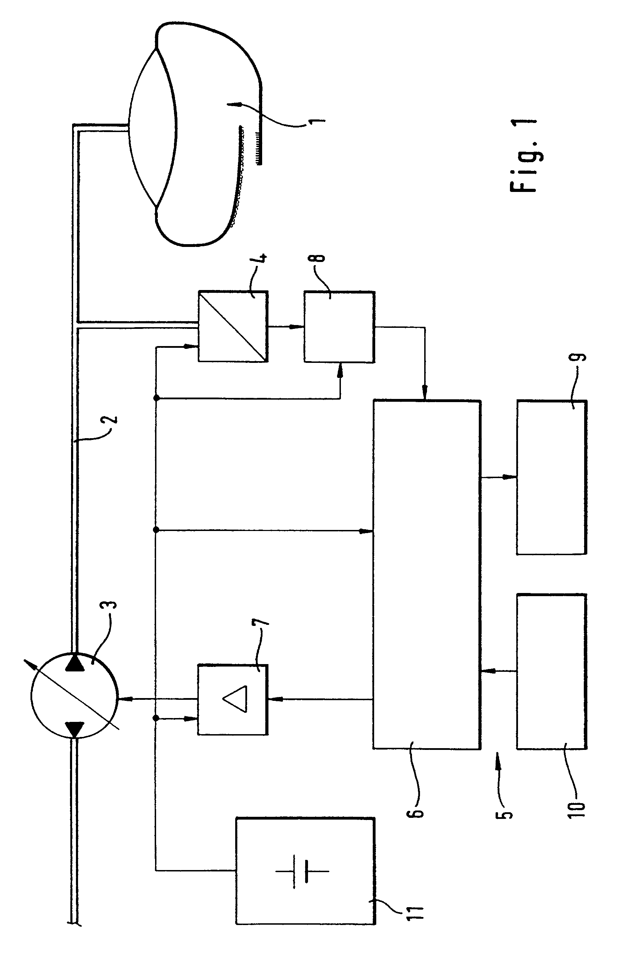

Das Blutdruckmeßgerät gemäß Figur 1 besitzt eine Manschette 1, die an ein Körperglied, insbesondere ein Handgelenk eines Benutzers angelegt und aufgepumpt werden kann. Hierzu besitzt die Manschette 1 einen Innenraum, der mit Fluid, insbesondere Luft, befüllt werden kann.

Mit dem Innenraum ist eine Fluidleitung 2 verbunden, die die Manschette 1 mit einer Pumpe 3 als Fördermittel verbindet. Die Pumpe 3 ist hinsichtlich ihres Durchsatzes bzw. ihrer Förderleistung einstellbar. Sie kann ein verstellbares Verdrängungsvolumen besitzen. Zum Antrieb der Pumpe ist ein in Figur 1 nicht gezeigter Gleichstrommotor vorgesehen.

Um die Pumpe 3 sowohl als Befüllorgan als auch als Auslaßorgan arbeiten lassen zu können, d. h. Fluid sowohl in die Manschette pumpen als auch Fluid aus der Manschette abfördern zu können und dadurch den Druck in der Manschette einzustellen, besitzt die Pumpe 3 zwei Förderrichtungen. Abhängig von der Drehrichtung des als Antrieb vorgesehenen Gleichstrommotors fördert die Pumpe 3 Fluid in einer ersten Förderrichtung in die Manschette 1 und fördert in einer zweiten Förderrichtung Fluid aus der Manschette 1 in entgegengesetzter Richtung ab. Die Drehrichtung des Gleichstrommotors kann sehr einfach durch die Polarität der elektrischen Spannung am Gleichstrommotor gesteuert werden. Zur Umpolung ist eine nicht näher dargestellte Umpoleinrichtung vorgesehen.

Um den in der Manschette herrschenden Fluiddruck erfassen zu können, ist eine Druckerfassungseinheit 4 vorgesehen, die mit dem Innenraum der Manschette 1 in Fluidverbindung steht. Die Druckerfassungseinheit 4 kann dabei grundsätzlich einen nach einem beliebigen Prinzip arbeitenden Drucksensor aufweisen. Vorzugsweise ist dies ein piezoresistiv arbeitender Drucksensor zur Erfassung des Drucks in der Manschette 1 und der Druckschwankungen bzw. -oszillationen infolge des Pulses.

Insbesondere bei einem Blutdruckmeßgerät mit einer Fingermanschette kann der Puls auch optisch erfaßt werden. Bei einem Finger liegen die Adern bedingt durch den geringen Durchmesser recht nahe an der Haut. Der Finger wird von außen z. B. mit einer Infrarotleuchtdiode angestrahlt. Aus der zeitlichen Veränderung der Signale eines zugehörigen Infrarotempfängers läßt sich ebenfalls die Volumenoszillation, d.h. zeitliche Schwankung der Blutmenge im Körperglied, detektieren. Der Vorteil der optischen Messung liegt darin, daß Störungen bzw. Druckschwankungen durch die Pulsation der Pumpe 3 auf das Signal des Drucksensors nicht die optische Messung beeinträchtigen.

Andererseits kann zur Reduzierung solcher Störungen auch eine in der Zeichnung nicht näher dargestellte Störfiltervorrichtung vorgesehen sein. Insbesondere kann dabei eine Erfassungsvorrichtung zur Erfassung der Betriebsfrequenz des Fördermittels und eine Frequenzfiltereinrichtung vorgesehen sein, die Signalanteile des von der Druckerfassungseinheit 4 bereitgestellten Drucksignals mit einer der Betriebsfrequenz der Pumpe 3 entsprechenden Frequenz filtert. Dies kann elektronisch z. B. analog oder in einem Microcontroller bewirkt werden. Es kann auch zwischen der Pumpe 3 und der Druckerfassungseinheit 4 in der Fluidleitung 2 ein an sich bekanntes mechanisches Dämpferglied zur Dämpfung von Druckschwankungen im Fluid vorgesehen sein.

Zur Steuerung der Pumpe 3 ist eine Steuereinheit 5 vorgesehen, die einen Microprozessor 6 aufweist. Die elektrische Steuereinheit 5 ist über einen Verstärker 7 mit der Pumpe 3 verbunden. Die Steuereinheit 5 steuert dabei zum einen die Förderrichtung der Pumpe 3, d. h. sie steuert die Umpoleinrichtung des Antriebs der Pumpe 3 an, und zum anderen die Förderleistung bzw. den Durchsatz der Pumpe 3, um die gewünschte Druckerhöhung bzw. -senkung in der Manschette 1 zu erzielen.

Die Steuereinheit 5 ist ferner mit der Druckerfassungseinheit 4 verbunden. Das Drucksignal der Druckerfassungseinheit 4 wird dabei über einen Analog/Digital-Wandler 8 der Steuereinheit 5 bereitgestellt. Die Steuereinheit kann dementsprechend die Pumpe 3 in Abhängigkeit des Drucksignals der Druckerfassungseinheit 4 steuern, um den erwünschten Manschettendruck zu bewirken. Darüber hinaus wird das Drucksignal der Druckerfassungseinheit 4 in einer Auswerteeinheit der Steuereinheit 5 ausgewertet, um den Blutdruck zu bestimmen, wie später noch näher erläutert werden wird.

Wie Figur 1 zeigt, ist die Steuereinheit 5 mit einer Ausgabeeinheit 9, beispielsweise einer Flüssigkristall-Anzeigeeinheit, zur Anzeige der Blutdruckwerte und mit einer Eingabeeinheit 10 zur Eingabe von Befehlen, wie beispielsweise dem Start der Messung, verbunden.

Zur Versorgung der jeweiligen Bauteile mit Energie, ist eine Stromquelle 11 vorgesehen, die insbesondere mit dem Verstärker 7, dem Microprozessor 6, der Druckerfassungseinheit 4 und dem Analog/Digital-Wandler 8 verbunden sein kann.

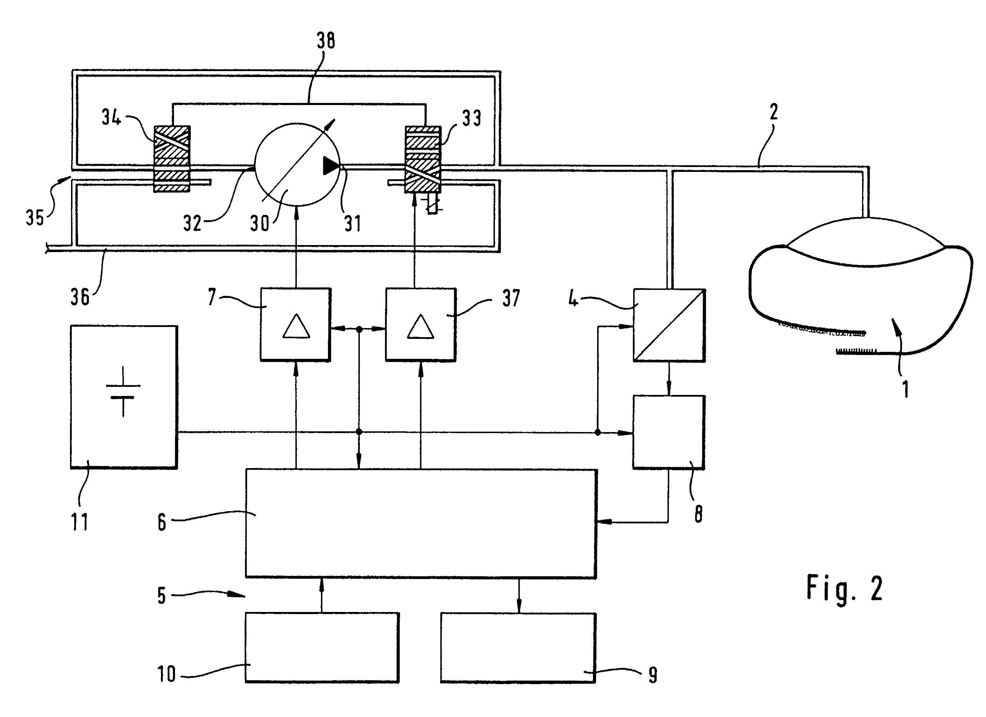

Vor der detaillierten Beschreibung der Funktion des Blutdruckmeßgerätes wird nachfolgend zunächst der Aufbau der zweiten Ausführungsform des Blutdruckmeßgerätes gemäß Figur 2 beschrieben. Diese Ausführungsform ist im Aufbau der in Figur 1 gezeigten grundsätzlich ähnlich, so daß für die gleichen Bauteile dieselben Bezugsziffern verwendet sind.

Diese Ausführung unterscheidet sich von der gemäß Figur 1 insbesondere darin, daß anstelle der bidirektionalen Pumpe 3 eine Pumpe 30 mit nur einer Förderrichtung vorgesehen ist. Die Pumpe 30 besitzt ebenfalls einen einstellbaren Durchsatz bzw. eine einstellbare Förderleistung.

Um die Förderrichtung der Pumpe 30 umkehren zu können, ist eine Umkehrvorrichtung 35 vorgesehen. Wie Figur 2 zeigt, besitzt die Pumpe 30 eine Pumpseite 31, zu der hin Fluid gepumpt wird, und eine Saugseite 32, an der Fluid angesaugt wird. Die Umkehrvorrichtung 35 weist ein Paar Schaltventile 33 und 34 auf, die an der Pumpseite 31 bzw. der Saugseite 32 angeordnet sind. Als Schaltventil kann dabei ein 3/2-Wege Ventil oder ein 4/2-Wege Ventil mit einem gesperrten Anschluß vorgesehen sein.

Das Schaltventil 33 verbindet dabei entsprechend seiner Schaltstellung die Pumpseite 31 der Pumpe 30 mit der zur Manschette 1 führenden Fluidleitung 2 oder mit einer Reservoirleitung 36, die mit einem Fluidreservoir oder im Falle, daß Luft als Fluid verwendet wird, mit der Umgebung verbunden ist. Das Schaltventil 34 verbindet entsprechend seiner Schaltstellung die Saugseite 32 ebenfalls entweder mit der Fluidleitung 2 oder mit der Reservoirleitung 36.

Die Umkehrvorrichtung 35 wird über einen Verstärker 37 von der Steuereinheit 5 angesteuert. Grundsätzlich kann die Steuereinheit jedes der beiden Schaltventile 33 und 34 separat ansteuern. Vorzugsweise jedoch erfolgt eine gemeinsame Ansteuerung. Die beiden Schaltventile 33 und 34 sind dabei in ihrer Schaltstellung über eine mechanische Kopplung 38 miteinander gekoppelt. Die beiden Schaltventile 33 und 34 sind dabei derart gekoppelt, daß niemals die Pumpseite 31 und die Saugseite 32 mit der Manschette 1 gleichzeitig in Fluidverbindung stehen. Insbesondere ist die Kopplung 38 derart ausgebildet, daß stets nur eine der beiden Seiten 31 und 32 mit der Manschette 1 in Fluidverbindung steht, während die andere Seite mit der Reservoirleitung 36 fluidverbunden ist.

In ihrer Ruhestellung, d. h. wenn die Schaltventile 33 und 34 von der Steuereinheit 5 nicht angesteuert sind, sind die beiden Schaltventile 33 und 34 derart geschaltet, daß die Manschette 1 mit der Saugseite 32 der Pumpe 30 und die Pumpseite 31 der Pumpe 30 mit der Reservoirleitung 36 fluidverbunden ist. Hierdurch kann auch bei einem Ausfall der Steuereinheit 5 der Druck in der Manschette 1 abgelassen werden.

Nachfolgend wird die Funktion und Wirkungsweise der die Erfindung verwirklichenden Blutdruckmeßgeräte näher erläutert.

Zunächst wird die Manschette 1 um eines der Handgelenke eines Benutzers gelegt. in einem nächsten Schritt wird das Blutdruckmeßgerät über die Eingabeeinheit 10 in Betrieb gesetzt. Die Steuereinheit 5 steuert dann die Pumpe 3 an. Dabei wird zunächst die Förderrichtung der Pumpe 3 durch Ansteuerung der Umpolvorrichtung derart festgelegt, daß die Pumpe Fluid in die Manschette 1 fördert. Im Falle des zweiten Ausführungsbeispieles gemäß Figur 2 steuert die Steuereinheit 5 die Umkehrvorrichtung 35 derart an, daß die Pumpseite 31 mit der Manschette 1 und die Saugseite 32 mit der Reservoirleitung 36 fluidverbunden ist.

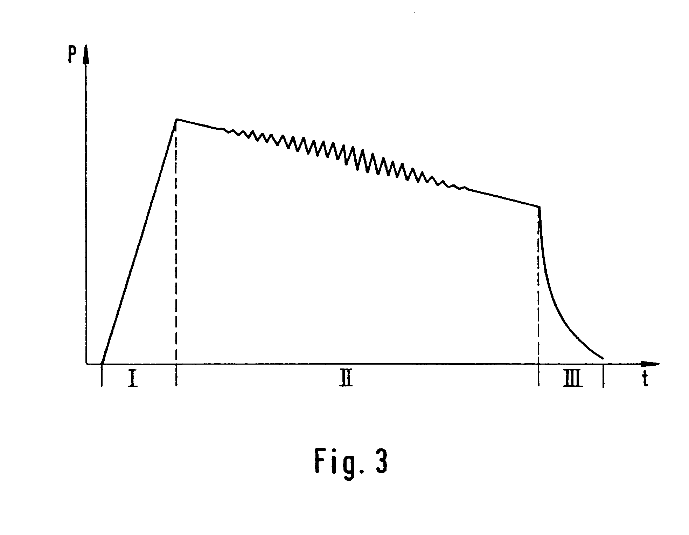

In einem weiteren Schritt wird die Pumpe 3 bzw. 30 mit einer hohen Förderleistung von der Steuereinheit 5 in Betrieb gesetzt, um in einem Aufpumpvorgang I (vgl. Figur 3) den Manschetteninnendruck rasch zu erhöhen, so daß die Arterie bis zur vollständigen Undurchlässigkeit für Blut zusammengedrückt ist und Blutdruckoszillationen verhindert sind. Sollten sich z.B. nach einem Aufpumpen der Manschette auf einen Anfangsdruck von 180 mm Hg sofort anschließend bereits Blutdruckoszillationen zeigen, so wird die Manschette solange intervallartig mit noch größerem Druck beaufschlagt bis die Arterie wirklich vollständig undurchlässig ist.

Daraufhin beginnt der eigentliche Meßvorgang, während dem der Druck in der Manschette 1 kontinuierlich gesenkt wird.

Um den Druck in der Manschette 1 zu reduzieren, polt die Steuereinheit 5 den Antrieb der Pumpe 3 um bzw. schaltet sie die Umkehrvorrichtung 35 in ihre Ruhestellung gemäß Figur 3, so daß die Saugseite 32 der Pumpe 30 mit der Manschette 1 verbunden ist und die Pumpseite 31 an die Reservoirleitung angeschlossen ist. Der Druck in der Manschette 1 wird dann in einer ersten Ablaßphase 11 mit einer langsamen Druckablaßrate vermindert (vgl. Figur 3). Dies kann zum einen durch Undichtigkeiten der Pumpe bewirkt bzw. unterstützt werden und zum anderen durch eine gezielte Betätigung der Pumpe 3 bzw. 30 durch die Steuereinheit 5 exakt gesteuert werden. Der jeweils tatsächlich herrschende Manschettendruck 1 wird dabei durch die Druckerfassungseinheit 4 erfaßt und von der Steuereinheit 5 zur Ansteuerung der Pumpe 3 bzw. 30 verarbeitet. Wie Figur 3 zeigt, erfolgt der Druckabfall während der langsamen Ablaßphase II mit einer in etwa konstanten Druckabfallrate, vorzugsweise von etwa 1 bis 10 mm Hg/s.

Bei einer zu schnellen Druckabsenkung, beispielsweise infolge einer zu großen Undichtigkeit der Pumpe, kann gegebenenfalls die Förderrichtung der Pumpe 3 bzw. 30 wieder umgekehrt werden, um dem raschen Druckabfall entgegenzusteuern.

Während des langsamen Druckabsenkens treten durch den Puls Oszillationen im Manschettendruck auf, wobei die Oszillationsamplitude zunächst zunimmt, ein Maximum erreicht und dann wieder abnimmt und gegen Null geht. Aus dem Oszillationsmaximum läßt sich dann bekanntermaßen der diastolische und der systolische Blutdruckwert bestimmen. Die Oszillation des Manschettendrucks kann dabei durch die Druckerfassungseinheit 4 erfaßt und in einer Auswerteeinheit der Steuereinheit 5 verarbeitet werden. Die Blutdruckwerte werden dann durch die Ausgabeeinheit 9 angezeigt. Wie erwähnt, kann die Vofumenoszillation auch durch einen optischen Sensor erfaßt werden.

In einem weiteren Schritt wird dann nach Erfassung der Oszillationen der Manschettendruck in einer schnellen Ablaßphase III mit einer großen Druckabfallrate rasch abgesenkt, um den Meßvorgang zu beschleunigen. Hierzu wird die Pumpe 3 bzw. 30 von der Steuereinheit 5 derart angesteuert, daß diese mit einer hohen Förderleistung Fluid aus der Manschette 1 abfördert. Vorzugsweise kann dabei die Ablaßzeit beim schnellen Ablassen III des Manschetteninnendrucks von einem anfänglichen Druck von etwa 260 mm Hg auf einen Druck von < 15 mm Hg auf eine Dauer von etwa 10 Sekunden oder weniger eingestellt werden, bzw. bei Geräten für Neugeborene und Kinder beim Ablassen von einem Druck von etwa 150 mm Hg auf einen Druck von < 5 mm Hg auf eine Periode von etwa 5 Sekunden eingestellt werden.

Sobald der Druck in der Manschette 1 abgelassen ist, schaltet die Steuereinheit 5 die Pumpe 3 bzw. 30 aus. Hierzu kann mit einem nicht in den Figuren gezeigten Strommesser der Eingangsstrom der Pumpe 3 bzw. 30 gemessen werden, der am Ende des Ablaßvorgangs ansteigt.

## Patentansprüche

1. Blutdruckmeßgerät mit einer fluidbefüllbaren Manschette (1) zum Anlegen an ein Körperglied, einer Befüllvorrichtung (2, 30, 35) zum Befüllen der Manschette (1) mit Fluid,
einer Auslaßvorrichtung (2, 30, 35) zum Auslassen des Fluids aus der Manschette (1), und einem Drucksensor (4) der derart in oder an die Manschette (1) gekoppelt ist, daß Druckoszillationen zur oszillometrischen Bestimmung des Blutdruckes meßbar sind, und wobei die Auslaßvorrichtung (2, 30, 35) ein antreibbares Fördermittel (30) zum Abfördern des Fluids aus der Manschette (1) aufweist,
**dadurch gekennzeichnet,**
**daß** das Fördermittel (30) eine Saugseite (32) und eine Pumpseite (31) aufweist und zwischen dem Fördermittel und der Manschette (1) eine Schaltvorrichtung (33, 34) vorgesehen ist, wobei in einer ersten Schaftstellung der Schaltvorrichtung die Saugseite des Fördermittels (30) und in einer zweiten Schaftstellung die Pumpseite des Fördermittels (30) mit der Manschette (1) fluidverbunden ist und wobei die Auslaßrvorrichtung (2, 30, 35) frei von geregelten Proportionalventilen ist.

2. Blutdruckmeßgerät nach Anspruch 1,
**dadurch gekennzeichnet,**
**daß** die Befüllvorrichtung (2, 30, 35) und die Auslaßvorrichtung (2, 30, 35) ein gemeinsames Fördermittel (3, 30) aufweisen.

3. Blutdruckmeßgerät nach Anspruch 1 oder 2,
**dadurch gekennzeichnet,**
**daß** das Fördermittel eine Pumpe (30) ist.

4. Blutdruckmeßgerät nach zumindest einem der vorhergehenden Ansprüche,
**dadurch gekennzeichnet,**
**daß** die Schaltvorrichtung zwei Schaltventile (33, 34) aufweist, die auf verschiedenen Seiten (31, 32) des Fördermittels (30) angeordnet sind und die derart miteinander gekoppelt sind, daß höchstens eine der Seiten des Fördermittels mit der Manschette (1) fluidverbunden ist.

5. Blutdruckmeßgerät nach zumindest einem der vorhergehenden Ansprüche,
**dadurch gekennzeichnet,**
**daß** eine Steuereinheit (5) zur Steuerung des Fördermittels (30) vorgesehen ist, wobei insbesondere die Steuereinheit mit einer Druckerfassungseinheit (4) zur Erfassung des Fluiddrucks in der Manschette (1) verbunden und zur Steuerung des Fördermittels in Abhängigkeit des erfaßten Fluiddrucks vorgesehen ist.

6. Blutdruckmeßgerät nach Anspruch 5,
**dadurch gekennzeichnet,**
**daß** das Fördermittel (30) von der Steuereinheit (5) derart ansteuerbar ist, daß der Fluiddruck in der Manschette (1) zumindest in einer Phase (II) des Ablaßvorganges mit einer konstanten Druckabfallrate vermindert wird, insbesondere derart, daß der Fluiddruck in einer ersten Phase (II) des Ablaßvorgangs mit einer ersten niedrigen Druckabfallrate und in einer zweiten Phase (III) mit einer zweiten hohen Druckabfallrate vermindert wird.

7. Blutdruckmeßgerät nach Anspruch 5 oder 6.
**dadurch gekennzeichnet,**
**daß** die Steuereinheit (5) eine Ablaß-Erfassungsvorrichtung zur Erfassung des Ablaßvorganges, insbesondere dessen Endes, aufweist, wobei vorzugsweise die Ablaß-Erfassungsvorrichtung einen Strommesser zur Messung eines Eingangsstromes des Fördermittels (30) aufweist.

8. Blutdruckmeßgerät nach zumindest einem der vorhergehenden Ansprüche,
**dadurch gekennzeichnet,**
**daß** eine Störfiltervorrichtung zur Filterung von Störungen in einem Manschettendrucksignal vorgesehen ist.

9. Blutdruckmeßgerät nach Anspruch 8,
**dadurch gekennzeichnet,**
**daß** die Störfiltervorrichtung eine Erfassungsvorrichtung zur Erfassung der Betriebsfrequenz des Fördermittels (30) und eine Frequenzfiltereinrichtung zur Filterung von Signalanteilen des Manschettendrucksignals mit einer der Betriebsfrequenz entsprechen den Frequenz aufweist.

10. Blutdruckmeßgerät nach Anspruch 8 oder 9,
**dadurch gekennzeichnet,**
**daß** die Störfiltervorrichtung zwischen dem Fördermittel (30) und einer Druckerfassungseinheit (4) zur Erfassung des Manschettendrucks ein Dämpferglied aufweist.

## Claims

1. A blood pressure measuring device having a cuff (1) adapted to be filled with fluid for application to a limb of a subject, a filling device (2, 30, 35) for filling the cuff (1) with fluid, a discharge device (2, 30, 35) for releasing the fluid from the cuff (1), and a pressure sensor (4) coupled in or to the cuff (1) in such fashion that pressure oscillations are measurable for the oscillometric determination of the blood pressure, said discharge device (2, 30, 35) comprising a drivable fluid delivery means (30) for discharging the fluid from the cuff (1),
**characterized in that** the fluid delivery means (30) has a suction side (32) and a discharge side (31), and that a switching device (33, 34) is provided between the fluid delivery means and the cuff (1), which switching device in a first switching position provides for fluid communication between the suction side of the fluid delivery means (30) and the cuff (1), and in a second switching position provides for fluid communication between the discharge side of the fluid delivery means (30) and the cuff (1), and said discharge device (2, 30, 35) being free from controlled proportioning valves.

2. The blood pressure measuring device as claimed in claim 1,
**characterized in that** the filling device (2, 30, 35) and the discharge device (2, 30, 35) have a common fluid delivery means (3, 30).

3. The blood pressure measuring device as claimed in claim 1 or 2,
**characterized in that** the fluid delivery means is a pump (30).

4. The blood pressure measuring device as claimed in at least one of the preceding claims,
**characterized in that** the switching device includes two switching valves (33, 34) arranged on different sides (31, 32) of the fluid delivery means (30) and coupled to each other so that at most one of the sides of the fluid delivery means is in fluid communication with the cuff (1) at a time.

5. The blood pressure measuring device as claimed in at least one of the preceding claims,
**characterized in that** provision is made for a control unit (5) for controlling the fluid delivery means (30), wherein in particular the control unit is connected to a pressure sensing unit (4) for detecting the fluid pressure in the cuff (1), and is constructed to control the fluid delivery means in response to the detected fluid pressure.

6. The blood pressure measuring device as claimed in claim 5,
**characterized in that** the fluid delivery means (30) is controllable by the control unit (5) in such a manner that the fluid pressure in the cuff (1) is reduced at a constant rate at least in one stage (II) of the deflation cycle, in particular in such a manner that in a first stage (II) of the deflation cycle the fluid pressure is reduced at a first low rate and in a second stage (III) at a second high rate.

7. The blood pressure measuring device as claimed in claim 5 or 6,
**characterized in that** the control unit (5) comprises a deflation sensing device for detecting the deflation cycle, in particular its end, wherein preferably the deflation sensing device includes a current measuring device for measuring an input current of the fluid delivery means (30).

8. The blood pressure measuring device as claimed in at least one of the preceding claims,
**characterized in that** provision is made for an interference filtering device for filtering out unwanted disturbances in a signal indicative of the cuff pressure.

9. The blood pressure measuring device as claimed in claim 8,
**characterized in that** the interference filtering device comprises a sensing device for detecting the operating frequency of the fluid delivery means (30) and a frequency filtering device for filtering signal components of the cuff pressure signal with a frequency corresponding to the operating frequency.

10. The blood pressure measuring device as claimed in claim 8 or 9,
**characterized in that** the interference filtering device comprises a damping element arranged between the fluid delivery means (30) and a pressure sensing device (4) for detecting the cuff pressure.

## Revendications

1. Appareil de mesure de la pression sanguine comprenant un manchon (1) pouvant être chargé par un fluide et destiné à être appliqué contre un organe corporel, un dispositif de remplissage (2, 30, 35) pour charger le manchon (1) avec un fluide, un dispositif de sortie (2, 30, 35) pour évacuer le fluide hors du manchon (1) et un capteur de pression (4) qui est couplé dans le ou au manchon (1) de telle sorte que des oscillations de pression peuvent être mesurées pour la détermination oscillométrique de la pression sanguine, et selon lequel le dispositif de sortie (2, 30, 35) comporte un moyen de convoyage (30) pouvant être entraîné pour évacuer le fluide hors du manchon (1),
**caractérisé en ce**
**que** le moyen de convoyage (30) comporte un côté aspiration (32) et un côté pompe (31) et qu'un dispositif de commutation (33, 34) est prévu entre le moyen de convoyage et le manchon (1), auquel cas dans une première position de commutation du dispositif de commutation, le côté aspiration du moyen de convoyage (30) est relié selon une liaison fluidique au manchon (1) et, dans une seconde position de commutation, le côté pompe du moyen de convoyage (30) est relié selon une liaison fluidique au manchon (1), et le dispositif de sortie (2, 30, 35) est exempt de soupapes réglées à action proportionnelle.

2. Appareil de mesure de la pression sanguine selon la revendication 1, **caractérisé en ce**
**que** le dispositif de remplissage (2, 30, 35) et le dispositif de sortie (2, 30, 35) comportent un moyen de convoyage commun (3, 30).

3. Appareil de mesure de la pression sanguine selon la revendication 1 ou 2, **caractérisé en ce que** le moyen de convoyage est une pompe (30).

4. Appareil de mesure de la pression sanguine selon au moins l'une des revendications précédentes, **caractérisé en ce que** le dispositif de commutation comporte deux soupapes de commutation (33, 34), qui sont disposées sur différents côtés (31, 32) du moyen de convoyage (30) et sont couplées entre elles de telle sorte qu'au maximum l'un des côtés du moyen de convoyage est relié selon une liaison fluidique au manchon (1).

5. Appareil de mesure de la pression sanguine selon au moins l'une des revendications précédentes, **caractérisé en ce qu'**une unité de commande (5) est prévue pour la commande du moyen de convoyage (30), et dans lequel notamment l'unité de commande est reliée à une unité de détection de pression (4) pour détecter la pression du fluide dans le manchon (1) et est prévue pour la commande du moyen de convoyage en fonction de la pression détectée du fluide.

6. Appareil de mesure de la pression sanguine selon la revendication 5, **caractérisé en ce que** le moyen de convoyage (30) peut être commandé par l'unité de commande (5) de telle sorte que la pression du fluide dans le manchon (1) est réduite au moins dans une phase (II) du processus d'évacuation avec un taux constant de réduction de la pression, notamment de telle sorte que la pression du fluide dans une première phase (II) du processus d'évacuation est réduite avec un premier taux faible de réduction de la pression et dans une seconde phase (III) avec un deuxième taux élevé de réduction de pression.

7. Appareil de mesure de la pression sanguine selon la revendication 5 ou 6, **caractérisé en ce que** l'unité de commande (5) comporte un dispositif de détection d'évacuation pour détecter le processus d'évacuation, notamment la fin du processus, auquel cas de préférence le dispositif de détection d'évacuation comporte un appareil de mesure de courant pour mesurer un courant d'entrée du moyen de convoyage (30).

8. Appareil de mesure de la pression sanguine selon au moins l'une des revendications précédentes, **caractérisé en ce qu'**il est prévu un dispositif de filtre des parasites pour filtrer les parasites dans un signal de pression du manchon.

9. Appareil de mesure de la pression sanguine selon la revendication 8, **caractérisé en ce que** le dispositif de filtre des parasites comporte un dispositif de détection servant à détecter la fréquence de fonctionnement du moyen de convoyage (30) et un dispositif formant filtre fréquentiel pour filtrer des composantes du signal de pression du manchon avec une fréquence correspondant à la fréquence de fonctionnement.

10. Appareil de mesure de la pression sanguine selon la revendication 8 ou 9, **caractérisé en ce que** le dispositif de filtre des parasites comprend un organe d'atténuation entre le moyen de convoyage (30) et une unité de détection de pression (4) pour détecter la pression du manchon.
